# EUROPEAN PATENT APPLICATION

(11) **EP 2 997 994 A1**
(43) Date of publication of application: **23.03.2016**
(21) Application number: 14185213.7
(22) Date of filing: 17.09.2014
(51) Int. Cl.: A61M 39/00, A61M 5/44, H05B 6/10, H05B 6/14

(54) **A hose assembly for medical fluid injection**

(71) Applicant: Sommer Udvikling & Medier IVS, 5220 Odense SØ (DK)
(72) Inventor: Praest, Mikkel, 5230 Odense M (DK); Sommer, Carsten, 5220 Odense SØ (DK)
(74) Representative: Plougmann Vingtoft a/s

(57) **Abstract**

There is provided a hose for use with medical fluids and, more particularly, to an induction heating assembly for use with the hose in administering heated medical fluids into living organisms. Such a hose has an induction heating. The susceptor receives a magnetic flux that generates heat, which is then transferred either directly to the medical fluids or to a conductive material that's receives the heat energy and conveys it along the longitudinal length of the specially designed hose. Therefore the heating of the medical fluid is not solely at the area where the heating assembly is located, but all along the hose. By using this principle, the hose heats the medical fluid more safely and with a much secure temperature control.

## Description

### FIELD OF THE INVENTION

The present invention generally relates to hoses for use with medical fluids and, more particularly, to hoses that can warm the medical fluids through induction heating for administering heated medical fluids into living organisms.

### BACKGROUND OF THE INVENTION

Administration of cold intravenous fluids and blood can produce substantial hypothermia, although the net effect of infusing cold solutions into the body depends on many factors such as tissue blood flow, rate of body heat generation, rate of heat loss to the outside environment, and temperature gradients within the body, age and concurrent illnesses.

A variety of commercial devices are available for warming intravenous fluids and blood. Many of these fluid warming devices do not deliver fluids at normothermia over a wide range of flows because of inefficient heat transfer of the warmer and heat loss along the length of the administrative tubing after the fluid exits the heat exchanger.

It is common to heat a physiological solution to normal body temperature before introducing it to the bloodstream intravenously. When the infusion rate is low, however, the heated solution often cools substantially before reaching the patient. One answer to this problem is to provide a patient line that is heated along its entire length by a warming fluid.

Many types of conduits have been proposed for heating a fluid or maintaining it at a predetermined temperature. Several of these conduits use one or more wires embedded in electrically insulating walls of the conduit to heat the fluid by electric resistance in the wires. While some of these devices are appropriate for their particular uses, they tend to heat in a linear, or local manner, resulting in local overheating of the fluid in the conduit. This effect is inconsequential in many applications.

Local overheating is reduced when the wiring is closely wound, but this leads to an increase in total resistivity and associated difficulties in controlling the amount of heating. Therefore, conduits containing tightly wound heating elements are unsuitable for use with physiological fluids, due to their inability to accurately maintain a fluid within a given temperature range. Furthermore, as with any embedded wire configuration, the heating density per output power of the wire at the heating wire surface is often so high that the wire becomes excessively heated, resulting in degradation of the surrounding wall material.

It is therefore an object of the present invention to provide a hose for medical fluids suitable for physiological uses which is flexible and at least partially transparent or translucent, and which is resistant to local overheating, chemical corrosion, thermal degradation and ensures normothemia medical fluid at the patient.

### SUMMARY OF THE INVENTION

In accordance with the present invention there is provided an induction heatable hose for medical fluid injection, comprising:
- A hose with a wall made of a polymeric material for placing in contact with the medical fluid;
- An induction heating susceptor disposed in the hose either located in the wall of the hose, the inner side of the hose or inside the hose as an integral part of the lumen of the hose, said induction heating susceptor may be covered with a plastic materiel being in direct contact with the medical fluid of the hose, and said induction-heating susceptor adapted to receive an induction signal from an exterior device;
- a heat conductor disposed in the hose either located in the wall of the hose or inside the hose as an integral part of the lumen in proximity of, or in direct contact with, the induction-heated susceptor, said heat conductor may be covered with a plastic materiel being in direct contact with the medical fluid of the hose, and said heat conductor adapted to receive heat from the induction-heated susceptor;
- optionally an insulated layer disposed in the hose either located in the wall of the hose, on the inner side the hose or on the outer side of the hose;
- optionally a first connector means for receiving said fluid from supply of physiological fluid; and
- optionally a second connector means for supplying the medical fluid to a patient.

In a preferred embodiment the induction-heating susceptor and the heat conductor are disposed inside the hose as an integral part of the lumen of the hose in the length direction of the hose, preferably as helically coiled. It is preferred that the induction-heating susceptor is made from iron. It is also preferred that the induction-heating susceptor is in direct contact with the heat conductor material, such as copper. The combination of the induction-heated susceptor and the conductor will henceforth be described as "the core".

In another aspect of the present invention there is provided a heating assembly adapted to fit the outer surface of the induction hose of the present invention. The assembly is provided with means for generating a magnetic flux so as to heat the induction heating susceptor. Preferably, the assembly comprises a temperature sensor for measuring the temperature of the medical fluid and a temperature control unit that regulates the magnetic flux to ensure a constant temperature of the medical fluid passing by in the hose. In a preferred embodiment the assembly covers 5-30 cm of the hose in the longitudinal direction.

In a particularly preferred embodiment the assembly, sandwiching the hose, is hinged in its longitudinal direction with a locking mechanism, such as a click lock, on the opposite side of the hinge.

To ensure constant medical fluid temperature the hose can be made with an insulating layer made which could consist of static air bubbles inside the polymeric material or a aluminium foil on the outer side of the hose wall but restricted to. The insulating layer also functions as a mitigation mechanism for interference due to temperature differences in the surrounding environment.

In a particularly preferred embodiment the assembly has a safety mechanism, which ensures the device can only be activated when a hose is inserted and the device is closed properly.

In a preferred embodiment the magnetic flux is induced by an alternate electric current having a predetermined electric power and a predetermined frequency causing the heating susceptor disposed inside the hose to heat the medical fluid to normal physiological temperature in the range of 35-43 C.

It is preferred that the temperature of the medical fluid within the hose of present invention is controlled through a software loop containing parameters of start temperature of the medical fluid, temperature of the surrounding environment, and induction effect. Medical fluid flow (ml/hr) may also be a parameter in such a control loop.

In another aspect of the present invention the hose and the heating assembly can be integrated as a singular unit, which can be connected to a standard intravenous assembly for administering medical fluids via LUERLOCK.

### BRIEF DESCRIPTION OF THE DRAWINGS

Figure 1 shows the core, where the heat conductor material is helically formed around the induction-heated susceptor.
Figure 2 shows a cross sections view of the medical fluid hose with the core disposed inside the hose as an integral part of the lumen of the hose as well as an insulating layer inside the hose wall.
Figure 3 shows a cross sections view of the medical fluid hose with the core located in the wall of the hose.
Figure 4 shows a cross sections view of the medical fluid hose with multiple cores disposed inside the hose as an integral part of the multiple lumen of the hose.
Figure 5 shows heating assembly adapted to fit the outer surface of the induction hose of the present invention.
Figure 6 shows a cross section view of said heating assembly.
Figure 7 shows an embodiment of the present invention where the induction coil of the heating assembly is an integral part of the hose and can be either the full length of the hose or a section of the hose.
Figure 8 shows the heating assembly with the locking mechanism.
Figure 9 shows an embodiment of the present invention where the hose and the heating assembly is a singular unit.

### DETAILED DESCRIPTION OF THE INVENTION

In the following a number of embodiments of the present invention are described in more detail.

The specially designed IV hose will receive the induction signal from the device, in its unique core inside, on the inner side of the specially IV hoses wall, or inside the lumen of the specially designed IV hose. The susceptor material can be iron but not restricted to. The susceptor material will be able to convert the induction signal into heat. This heat will be transferred to a heat conductor material directly connected with or in proximity of the susceptor material. This conductor material can be made of copper but not restricted to. These two materials can be made into a rod, wire, alloy, string or plate but not restricted to. The conducted heat will move throughout the length of the core inside the hose, and are therefore not restricted to only heat at the site of the device itself. To protect against unnecessary heat loss, the hose can be constructed with an insulating layer on the outer side, the inside or on the inner side of the hose wall. The insulating layer can be a thin layer of static air or aluminium foil but not restricted to.

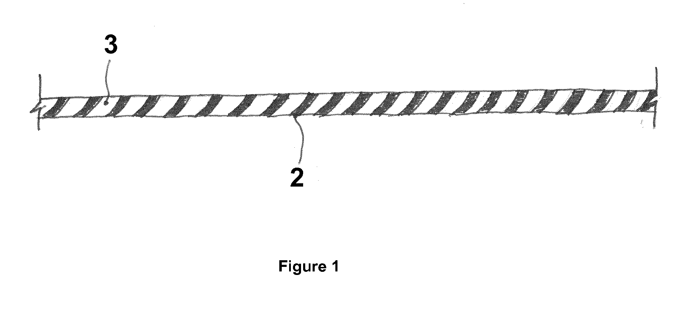

Referring to Figure 1 the core **1** is made by helically forming the heat conductor material **2** around the induction susceptor material **3.**

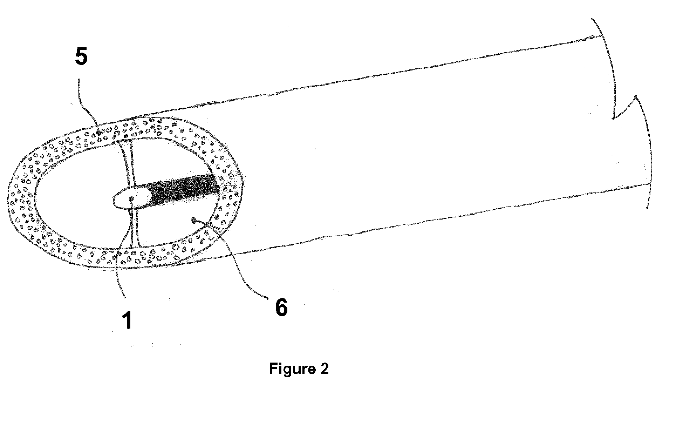

In Figure 2 the IV hose **4** is shown with the fully integrated core **1**' disposed in the central part of the hose (length direction). Also shown in Figure 2 is an insulating layer **5** in the wall of the IV hose **4.**

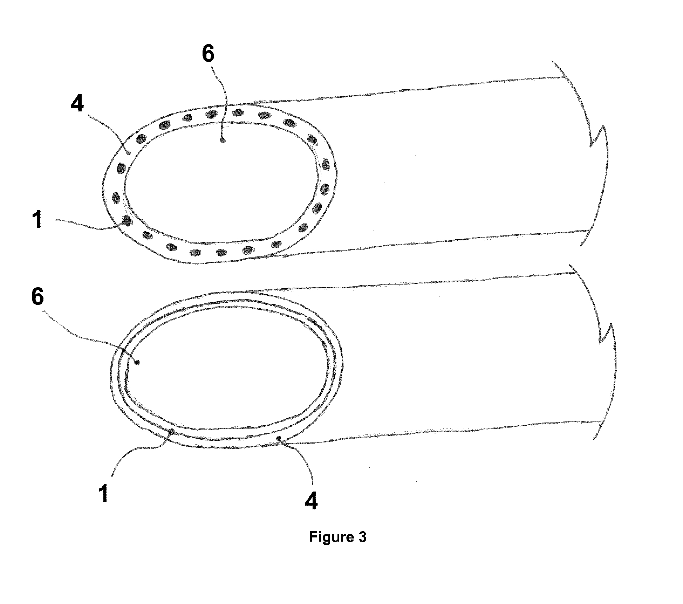

Figure 3 shows the IV hose **4** of the present invention is shown with the core **1"** disposed inside the wall of the hose depicted here as multiple rods or a cylinder in the length direction.

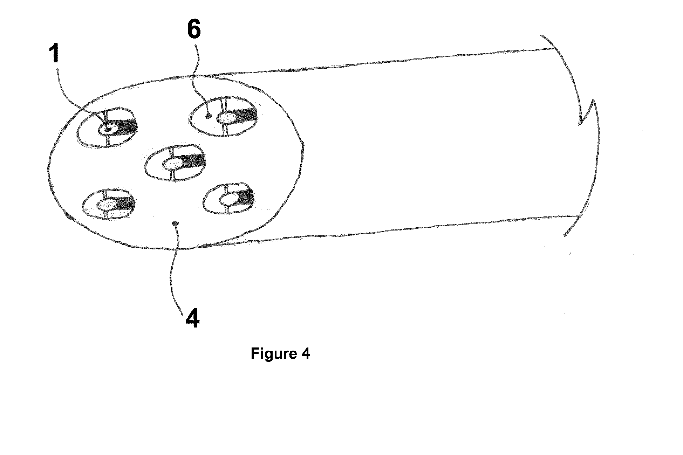

As it appears in Figure 4, the present invention can be constructed with multiple lumina for containing medical fluids **6** with each lumen having its own core **1"'.**

The IV hose **4** is designed with a lumen to contain the medical fluid **6,** and deliver it directly into the intravenous access in the patients and a Luerlock system **7** at either end of the hose.

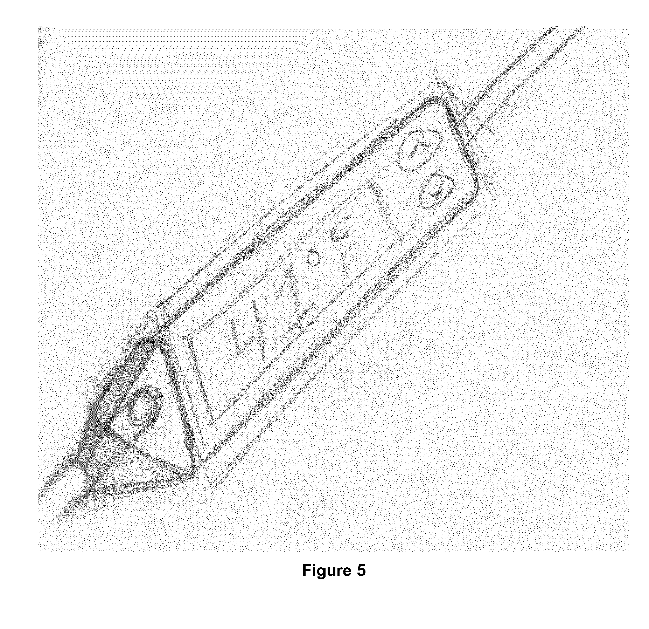

As appears from Figure 5 the present invention also provides a heating assembly **8** adapted to fit the outer surface of the IV hose **4** of the present invention. The assembly is provided with means for generating a magnetic flux so as to heat the induction susceptor material 3. Preferably, the assembly comprises a temperature sensor for measuring the temperature of the medical fluid and a temperature control unit that regulates the magnetic flux to ensure a constant temperature of the medical fluid flowing in the IV hose **4.** In a preferred embodiment the assembly covers 5-30 cm of the IV hose **4** in the longitudinal direction and regulates the medical fluid temperature in a minimum of 1 metre length of the IV hose **4.**

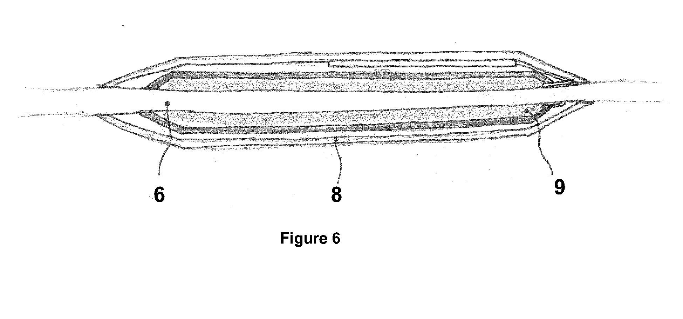

Figure 6 show a cross section of the heating assembly **8** where the inductive coils **9** are wrapped tightly for maximum induction efficiency.

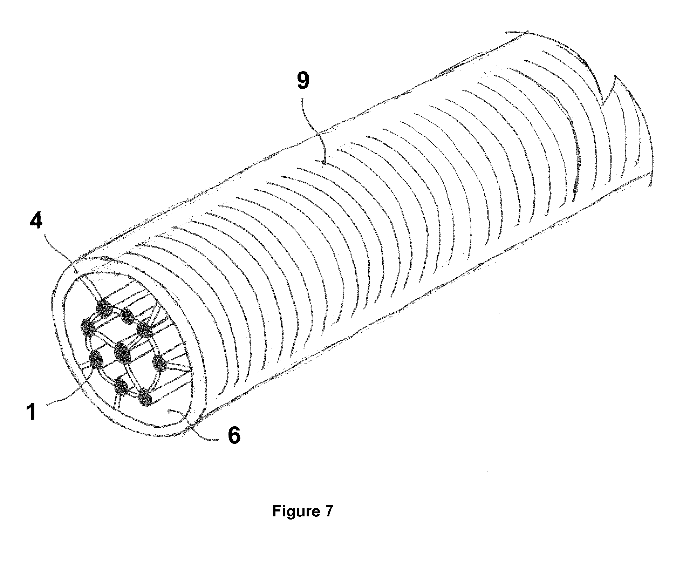

Another aspect of the present invention is shown in Figure 7, where the induction coils **9** are integrated in the IV hose 4 and the rest of the heating assembly 8 (see Figure 6) is located off site, preferably along the rest of the electronic equipment. External wiring connects the induction coils **9** and the heating assembly **8.**

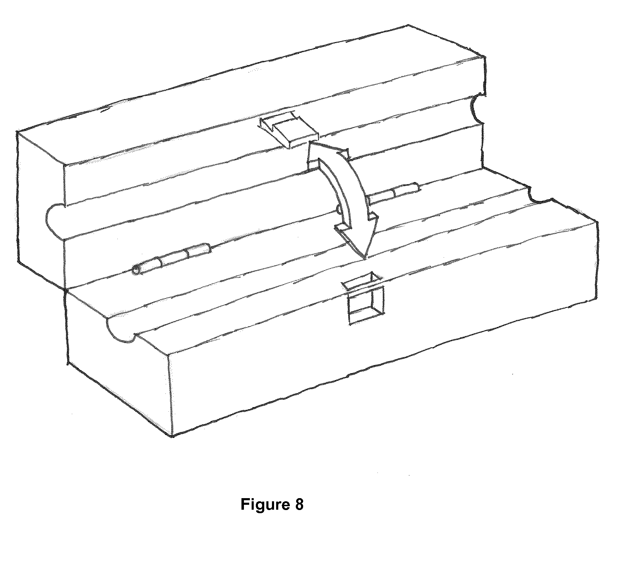

The primary device will make an induction signal designed for a specially designed IV hose. The device form will be small and with low weight, under 1 kg. The device will be able to fit the specially designed IV hose in its inner lumen, after it has been closed. By introducing the induction signal towards the specially designed IV hose the device will transfer energy to the IV hose. The shape of the device can but is not restricted to the option in the attached picture. The device will be able to be opened or closed by the user by a simple click (see Figure 8).

This software of the heating assembly uses either a manually programmed or automatically pre-programmed of desired temperature in which the medical fluid should be administrated, then measures temperature from the temperature sensor an applies it in a magnetic flux control loop to establish a constant temperature of the fluid. This software loop is continuously rechecked via a sensor control interval system in pre-programmed intervals, thus ensuring a safe heating of the medical fluid.

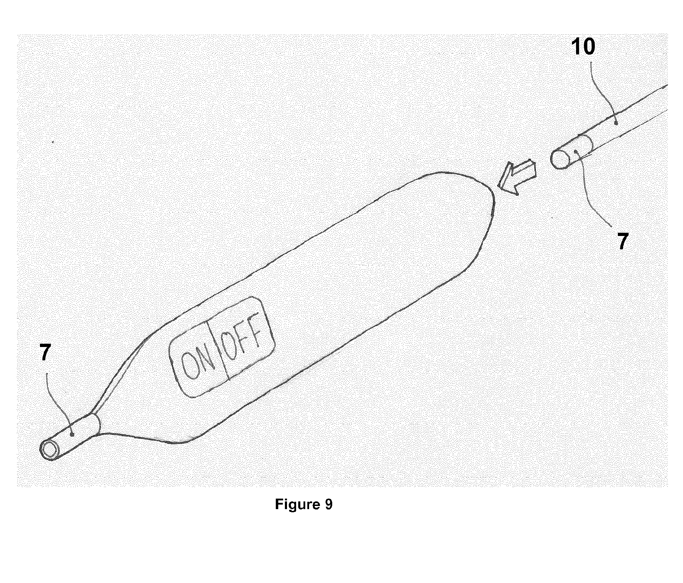

The present invention and the heating assembly can be manufactured to one singular unit, shown in Figure 9, where specially designed lumen to contain medical fluid 6 is moulded to optimise the heating efficiency. Such a singular unit would have Luerlock system **7** connecting it to a normal intravenous medical fluid administering system **10.**

### References:

1. Core
2. Conductor material
3. Susceptor material
4. IV hose
5. Insulating layer
6. Lumen to contain the medical fluid
7. Luerlock system
8. Heating assembly
9. Inductive coils
10. Intravenous medical fluid administering system

## Claims

1. An induction heatable hose for medical fluid injection, comprising:
• a hose with a wall made of a polymeric material for placing in contact with the medical fluid;
• an induction heating susceptor disposed in the hose either located in the wall of the hose, the inner side of the hose or inside the hose as an integral part of the lumen of the hose, said induction-heating susceptor optionally covered with a plastic materiel being in direct contact with the medical fluid of the hose, and said induction-heating susceptor adapted to receive an induction signal from an exterior device;
• a heat conductor disposed in the hose either located in the wall of the hose or inside the hose as an integral part of the lumen in proximity of, or in direct contact with, the induction-heated susceptor, said heat conductor optionally covered with a plastic materiel being in direct contact with the medical fluid of the hose, and said heat conductor adapted to receive heat from the induction-heated susceptor;
• optionally an insulated layer disposed in the hose either located in the wall of the hose, on the inner side the hose or on the outer side of the hose;
• optionally a first connector means for receiving said fluid from supply of physiological fluid; and
• optionally a second connector means for supplying the medical fluid to a patient.

2. The induction heatable hose of claim 1, wherein the induction-heating susceptor is disposed inside the hose as an integral part of the lumen of the hose in the length direction of the hose, preferably as a helically coiled susceptor.

3. The induction heatable hose of claim 1 or 2, wherein the induction-heating susceptor is made from iron.

4. The induction heatable hose of any one of the claims 1-3, wherein the induction-heating susceptor is covered with a heat conductive material.

5. The induction heatable hose of claim 4, wherein the heat conductive material is copper.

6. The induction heatable hose of any one of the claims 1-5, wherein the induction-heating susceptor and the heat conductive material are formed together or separate as a rod, wire, alloy, string or plate.

7. A heating assembly adapted to fit the outer surface of the induction hose of any one of the claims 1-6 said assembly provided with means for generating a magnetic flux to heat the induction heating susceptor.

8. The heating assembly of claim 7, wherein the assembly comprises a temperature sensor for measuring the temperature of the medical fluid and a temperature control unit that regulates the magnetic flux to ensure a constant temperature of the medical fluid flowing in the hose.

9. The heating assembly of claim 7 or 8, wherein the assembly covers 5-30 cm of the hose in the longitudinal direction.

10. The heating assembly of any one of the claims 1-9, wherein the assembly sandwiching the hose is hinged in its longitudinal direction with a locking mechanism on the opposite side of the hinge.

11. The heating assembly of claim 10, wherein the locking mechanism is a click lock enabling the assembly to be opened or closed by a user by a simple click.
